(19) Europäisches Patentamt
European Patent Office
Office européen des brevets

(11) EP 2 198 853 B1

(12) **FASCICULE DE BREVET EUROPEEN**

(45) Date de publication et mention de la délivrance du brevet:
**12.08.2015 Bulletin 2015/33**

(21) Numéro de dépôt: **09179884.3**

(22) Date de dépôt: **18.12.2009**

(51) Int Cl.:
***A61K 8/92*** (2006.01) ***A61K 8/31*** (2006.01)
***A61K 8/34*** (2006.01) ***A61K 8/37*** (2006.01)
***A61K 8/41*** (2006.01) ***A61Q 5/10*** (2006.01)

(54) **Composition de teinture d'oxydation des fibres kératiniques comprenant un corps gras et la n, n bis (beta-hydroxyéthyl)-paraphénylène diamine**

Oxidationsfärbemittel für keratiniche Fasern enthaltend einen Fettkörper und N,N bis-(beta-hydroxyethyl)-paraphenylenediamine

Composition for the oxidative dyeing of keratinous fibres comprising a fatty material and N,N bis-(beta-hydroxyethyl)-paraphenylenediamine

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **19.12.2008 FR 0807319**

(43) Date de publication de la demande:
**23.06.2010 Bulletin 2010/25**

(73) Titulaire: **L'Oréal**
**75008 Paris (FR)**

(72) Inventeurs:
- **Audousset, Marie-Pascale**
**92600, Asnieres (FR)**
- **Schlosser, Isabelle**
**75009, Paris (FR)**

(74) Mandataire: **Fevrier, Murielle Françoise E.**
**L'Oréal**
**River Plaza - DIPI**
**25-29 Quai Aulagnier**
**92665 Asnières-sur-Seine (FR)**

(56) Documents cités:
**WO-A-01/60327**
**DE-A1-102006 012 575**
**FR-A- 2 779 949**
**JP-A- 10 101 537**
**JP-A- 2003 238 370**
**US-B1- 6 238 653**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 198 853 B1

Printed by Jouve, 75001 PARIS (FR)

## Description

La présente demande a pour objet une composition pour la teinture d'oxydation des fibres kératiniques.

Il est connu de teindre les fibres kératiniques et en particulier les cheveux humains avec des compositions tinctoriales contenant des colorants d'oxydation, en particulier des précurseurs de colorants d'oxydation et des modificateurs de coloration.

Les précurseurs de colorants d'oxydation, appelés généralement bases d'oxydation, sont initialement des composés incolores ou faiblement colorés qui, associés à des produits oxydants, peuvent donner naissance par un processus de condensation oxydative à des composés colorés et colorants. Ce sont en général des composés tels que les ortho- ou para-phénylènediamines, les ortho- ou para-aminophénols et les bases hétérocycliques.

On sait également que l'on peut faire varier les nuances obtenues avec ces bases d'oxydation en les associant à des coupleurs ou modificateurs de coloration, ces derniers étant généralement choisis parmi les méta-diaminobenzènes, les méta-aminophénols, les méta-diphénols et certains composés hétérocycliques tels que des composés indoliques.

La variété des molécules mises en jeu au niveau des bases d'oxydation et des coupleurs permet l'obtention d'une riche palette de couleurs.

La coloration dite "permanente" obtenue grâce à ces colorants d'oxydation, appelée également coloration d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agressions extérieures telles que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui comporte en général des zones différemment sensibilisées (c'est-à-dire abîmées) de sa pointe à sa racine.

De nombreuses tentatives ont été faites dans le domaine de la coloration capillaire afin d'améliorer les propriétés tinctoriales à l'aide, par exemple, d'adjuvants. Cependant, le choix de ces adjuvants est délicat dans la mesure où ils doivent améliorer les propriétés tinctoriales des compositions tinctoriales sans nuire aux autres propriétés de ces compositions. En particulier, ces adjuvants ne doivent pas nuire aux propriétés d'éclaircissement des fibres kératiniques et les propriétés d'application de la coloration.

Le but de la présente invention est d'obtenir de nouvelles compositions pour la teinture d'oxydation des fibres kératiniques qui ne présentent pas les inconvénients de l'art antérieur. Plus particulièrement, le but de la présente invention est d'obtenir des compositions de coloration d'oxydation des fibres kératiniques, présentant des propriétés tinctoriales améliorées et qui soient faciles à mélanger et à appliquer, notamment qui ne coulent pas et restent bien localisées au point d'application. Par propriétés tinctoriales améliorées, on entend en particulier une amélioration au niveau de la puissance/intensité et/ou de l'homogénéité de la teinture.

Ainsi, ce but est atteint par la présente invention qui a pour objet une composition pour la teinture d'oxydation des fibres kératiniques, et en particulier des fibres kératiniques humaines telles que les cheveux, comprenant,

A) au moins 25 % de corps gras, différents des acides gras
B) une ou plusieurs bases d'oxydation choisies parmi la N,N β-hydroxyéthyl paraphénylène diamine et les sels d'addition avec un acide correspondants,
C) un ou plusieurs précurseurs de colorant additionnels différents de la base d'oxydation définie sous B),
D) un ou plusieurs agents oxydants et éventuellement
E) un ou plusieurs agents alcalins.

La composition conforme à la présente invention se distingue par ses propriétés tinctoriales améliorées. En particulier, la composition de l'invention conduit à des colorations qui présentent une bonne puissance et/ou intensité et/ou une bonne homogénéité de la couleur le long de la fibre entre la pointe et la racine des cheveux (appelé aussi la sélectivité de la coloration) et/ou une bonne chromaticité. La composition de l'invention s'applique sans difficulté sur les fibres kératiniques, sans couler. Cette composition permet aussi une plus faible dégradation des fibres kératiniques au cours du processus de coloration.

Enfin, les colorations obtenues à l'aide des compositions de l'invention sont tenaces, et résistent aux diverses agressions extérieures que peuvent subir les fibres kératiniques.

La présente invention a également pour objet un procédé de teinture des fibres kératiniques mettant en oeuvre la composition conforme à l'invention.

La présente invention a aussi pour objet un dispositif à plusieurs compartiments pour la mise en oeuvre de la composition de l'invention.

La présente invention a pour objet l'utilisation pour la teinture d'oxydation des fibres kératiniques de la composition conforme à l'invention.

Ainsi que cela a été mentionné, la composition de l'invention comprend un ou plusieurs corps gras.

Par corps gras, on entend, un composé organique insoluble dans l'eau à température ordinaire (25°C) et à pression atmosphérique (760 mm de Hg) (solubilité inférieure à 5% et de préférence à 1% encore plus préférentiellement à 0,1%). Ils présentent dans leur structure un enchaînement d'au moins deux groupements siloxane ou au moins une chaine hydrocarbonée comportant au moins 6 atomes de carbone. En outre, les corps gras sont généralement solubles dans les solvants organiques dans les mêmes conditions de température et de pression, comme par exemple le chloroforme, l'éthanol, le benzène ou le décaméthylcyclopentasiloxane.

Selon l'invention, les corps gras sont choisis parmi les corps gras différents des acides gras.

Les corps gras sont notamment choisis parmi les alcanes inférieurs, les alcools gras, les esters d'acide gras, les esters d'alcool gras, les huiles en particulier les huiles non siliconées minérales, végétales, animales ou synthétiques, les cires non siliconées et les silicones.

Il est rappelé qu'au sens de l'invention, les alcools, esters et acides gras présentent plus particulièrement un ou plusieurs groupements hydrocarbonés, linéaires ou ramifiés, saturés ou insaturés, comprenant 6 à 30 atomes de carbone, éventuellement substitués, en particulier par un ou plusieurs groupements hydroxyle (en particulier 1 à 4). S'ils sont insaturés, ces composés peuvent comprendre une à trois double-liaisons carbone-carbone, conjuguées ou non.

En ce qui concerne les alcanes inférieurs, ces derniers comprennent de préférence de 6 à 16 atomes de carbone et sont linéaires ou ramifiés, éventuellement cycliques. A titre d'exemple, les alcanes peuvent être choisis parmi l'hexane et le dodécane, les isoparaffines comme l'isohexadécane et l'isodécane.

Comme huiles non siliconées utilisables dans la composition de l'invention, on peut citer par exemple :

- les huiles hydrocarbonées d'origine animale, telles que le perhydrosqualène ;
- les huiles hydrocarbonées d'origine végétale, telles que les triglycérides liquides d'acides gras comportant de 6 à 30 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol® 810, 812 et 818 par la société Dynamit Nobel, l'huile de jojoba, l'huile de beurre de karité ;
- les hydrocarbures de plus de 16 atomes de carbone linéaires ou ramifiés, d'origine minérale ou synthétique, tels que les huiles de paraffine, la vaseline, l'huile de vaseline, les polydécènes, les polyisobutènes hydrogénés tel que Parléam®.
- les huiles fluorées partiellement hydrocarbonées ; comme huiles fluorées, on peut citer aussi le perfluorométhylcyclopentane et le perfluoro-1,3 diméthylcyclohexane, vendus sous les dénominations de "FLUTEC® PC1" et "FLUTEC® PC3" par la Société BNFL Fluorochemicals ; le perfluoro-1,2-diméthylcyclobutane ; les perfluoroalcanes tels que le dodécafluoropentane et le tétradécafluorohexane, vendus sous les dénominations de "PF 5050®" et "PF 5060®" par la Société 3M, ou encore le bromoperfluorooctyle vendu sous la dénomination "FORALKYL®" par la Société Atochem ; le nonafluoro-méthoxybutane et le nonafluoroéthoxyisobutane ; les dérivés de perfluoromorpholine, tels que la 4-trifluorométhyl perfluoromorpholine vendue sous la dénomination "PF 5052®" par la Société 3M.

Les alcools gras utilisables comme corps gras dans la composition de l'invention sont non oxyalkylénés, saturés ou insaturés, linéaires ou ramifiés, et comportent 6 à 30 atomes de carbone et plus particulièrement de 8 à 30 atomes de carbone, on peut citer l'alcool cétylique, l'alcool stéarylique et leur mélange (alcool cétylstéarylique), l'octyldodécanol, le 2- butyloctanol, le 2-hexyldécanol, le 2-undécylpentadécanol, l'alcool oléique ou l'alcool linoléique.

La cire ou les cires non siliconées susceptibles d'être utilisées dans la composition de l'invention sont choisies notamment, parmi la cire de Carnauba, la cire de Candelila, et la cire d'Alfa, la cire de paraffine, l'ozokérite, les cires végétales comme la cire d'olivier, la cire de riz, la cire de jojoba hydrogénée ou les cires absolues de fleurs telles que la cire essentielle de fleur de cassis vendue par la société BERTIN (France), les cires animales comme les cires d'abeilles, ou les cires d'abeilles modifiées (cerabellina) ; d'autres cires ou matières premières cireuses utilisables selon l'invention sont notamment les cires marines telles que celle vendue par la Société SOPHIM sous la référence M82, les cires de polyéthylène ou de polyoléfines en général.

Les esters sont les esters de mono ou polyacides aliphatiques saturés ou insaturés, linéaires ou ramifiés en $C_1$-$C_{26}$ et de mono ou polyalcools aliphatiques saturés ou insaturés, linéaires ou ramifiés en $C_1$-$C_{26}$, le nombre total de carbone des esters étant plus particulièrement supérieur ou égal à 10.

Parmi les monoesters, on peut citer le béhénate de dihydroabiétyle ; le béhénate d'octyldodécyle ; le béhénate d'isocétyle ; le lactate de cétyle ; le lactate d'alkyle en $C_{12}$-$C_{15}$ ; le lactate d'isostéaryle ; le lactate de lauryle ; le lactate de linoléyle ; le lactate d'oléyle ; l'octanoate de (iso)stéaryle ; l'octanoate d'isocétyle ; l'octanoate d'octyle ; l'octanoate de cétyle ; l'oléate de décyle ; l'isostéarate d'isocétyle ; le laurate d'isocétyle ; le stéarate d'isocétyle ; l'octanoate

d'isodécyle ; l'oléate d'isodécyle ; l'isononanoate d'isononyle ; le palmitate d'isostéaryle ; le ricinoléate de méthyle acétyle ; le stéarate de myristyle ; l'isononanoate d'octyle ; l'isononate de 2-éthylhexyle ; le palmitate d'octyle ; le pélargonate d'octyle ; le stéarate d'octyle ; l'érucate d'octyldodécyle ; l'érucate d'oléyle ; les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, de mirystyle, de stéaryle le stéarate d'hexyle, le stéarate de butyle, le stéarate d'isobutyle ; le malate de dioctyle, le laurate d'hexyle, le laurate de 2-hexyldécyle.

Toujours dans le cadre de cette variante, on peut également utiliser les esters d'acides di ou tricarboxyliques en $C_4$-$C_{22}$ et d'alcools en $C_1$-$C_{22}$ et les esters d'acides mono di ou tricarboxyliques et d'alcools di, tri, tétra ou pentahydroxy en $C_2$-$C_{26}$.

On peut notamment citer : le sébacate de diéthyle ; le sébacate de diisopropyle ; l'adipate de diisopropyle ; l'adipate de di n-propyle ; l'adipate de dioctyle ; l'adipate de diisostéaryle ; le maléate de dioctyle ; l'undecylénate de glycéryle ; le stéarate d'octyldodécyl stéaroyl ; le monoricinoléate de pentaérythrityle ; le tétraisononanoate de pentaérythrityle ; le tétrapélargonate de pentaérythrityle ; le tétraisostéarate de pentaérythrityle ; le tétraoctanoate de pentaérythrityle ; le dicaprylate de propylène glycol ; le dicaprate de propylène glycol, l'érucate de tridécyle ; le citrate de triisopropyle ; le citrate de triisotéaryle ; trilactate de glycéryle ; trioctanoate de glycéryle ; le citrate de trioctyldodécyle ; le citrate de trioléyle, le dioctanoate de propylène glycol ; le diheptanoate de néopentyl glycol ; le diisanonate de diéthylène glycol ; et les distéarates de polyéthylène glycol.

Parmi les esters cités ci-dessus, on préfère utiliser les palmitates d'éthyle, d'isopropyle, de myristyle, de cétyle, de stéaryle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle et l'isononanate d'isononyle, l'octanoate de cétyle.

La composition peut également comprendre, à titre d'ester gras, des esters et di-esters de sucres d'acides gras en $C_6$-$C_{30}$, de préférence en $C_{12}$-$C_{22}$. Il est rappelé que l'on entend par « sucre », des composés hydrocarbonés oxygénés qui possèdent plusieurs fonctions alcool, avec ou sans fonction aldéhyde ou cétone, et qui comportent au moins 4 atomes de carbone. Ces sucres peuvent être des monosaccharides, des oligosaccharides ou des polysaccharides.

Comme sucres convenables, on peut citer par exemple le sucrose (ou saccharose), le glucose, le galactose, le ribose, le fucose, le maltose, le fructose, le mannose, l'arabinose, le xylose, le lactose, et leurs dérivés notamment alkylés, tels que les dérivés méthylés comme le méthylglucose.

Les esters de sucres et d'acides gras peuvent être choisis notamment dans le groupe comprenant les esters ou mélanges d'esters de sucres décrits auparavant et d'acides gras en $C_6$-$C_{30}$, de préférence en $C_{12}$-$C_{22}$, linéaires ou ramifiés, saturés ou insaturés. S'ils sont insaturés, ces composés peuvent comprendre une à trois double-liaisons carbone-carbone, conjuguées ou non.

Les esters selon cette variante peuvent être également choisis parmi les mono-, di-, tri- et tétra-esters, les polyesters et leurs mélanges.

Ces esters peuvent être par exemple des oléate, laurate, palmitate, myristate, béhénate, cocoate, stéarate, linoléate, linolénate, caprate, arachidonates, ou leurs mélanges comme notamment les esters mixtes oléo-palmitate, oléo-stéarate, palmito-stéarate.

Plus particulièrement, on utilise les mono- et di- esters et notamment les mono- ou di- oléate, stéarate, béhénate, oléopalmitate, linoléate, linolénate, oléostéarate, de saccharose, de glucose ou de méthylglucose.

On peut citer à titre d'exemple le produit vendu sous la dénomination Glucate® DO par la société Amerchol, qui est un dioléate de méthylglucose.

On peut aussi citer à titre d'exemples d'esters ou de mélanges d'esters de sucre d'acide gras :

- les produits vendus sous les dénominations F160, F140, F110, F90, F70, SL40 par la société Crodesta, désignant respectivement les palmito-stéarates de sucrose formés de 73% de monoester et 27% de di- et tri-ester, de 61% de monoester et 39% de di-, tri-, et tétra-ester, de 52% de monoester et 48 % de di-, tri-, et tétra-ester, de 45% de monoester et 55% de di-, tri-, et tétra-ester, de 39% de monoester et 61% de di-, tri-, et tétra-ester, et le monolaurate de sucrose;
- les produits vendus sous la dénomination Ryoto Sugar Esters par exemple référencés B370 et correspondant au béhénate de saccharose formé de 20% de monoester et 80 % de di-triester-polyester;
- le mono-di-palmito-stéarate de sucrose commercialisé par la société Goldschmidt sous la dénomination Tegosoft® PSE.

Les silicones utilisables dans la composition de la présente invention, sont des silicones volatiles ou non volatiles, cycliques, linéaires ou ramifiées, modifiées ou non par des groupements organiques, ayant une viscosité de 5.10-6 à 2,5m2/s à 25°C et de préférence 1.10-5 à 1m2/s.

Les silicones utilisables conformément à l'invention peuvent se présenter sous forme d'huiles, de cires, de

résines ou de gommes.

De préférence, la silicone est choisie parmi les polydialkylsiloxanes, notamment les polydiméthylsiloxanes (PDMS), et les polysiloxanes organo-modifiés comportant au moins un groupement fonctionnel choisi parmi les groupements poly(oxyalkylène), les groupements aminés et les groupements alcoxy.

Les organopolysiloxanes sont définis plus en détail dans l'ouvrage de Walter NOLL "Chemistry and Technology of Silicones" (1968), Academie Press. Elles peuvent être volatiles ou non volatiles.

Lorsqu'elles sont volatiles, les silicones sont plus particulièrement choisies parmi celles possédant un point d'ébullition compris entre 60°C et 260°C, et plus particulièrement encore parmi:

les polydialkylsiloxanes cycliques comportant de 3 à 7, de préférence de 4 à 5 atomes de silicium. Il s'agit, par exemple, de l'octaméthylcyclotétrasiloxane commercialisé notamment sous le nom de VOLATILE SILICONE® 7207 par UNION CARBIDE ou SILBIONE® 70045 V2 par RHODIA, le décaméthylcyclopentasiloxane commercialisé sous le nom de VOLATILE SILICONE® 7158 par UNION CARBIDE, et SILBIONE® 70045 V5 par RHODIA, ainsi que leurs mélanges.

On peut également citer les cyclocopolymères du type diméthylsiloxanes/méthylalkylsiloxane, tel que la SILICONE VOLATILE® FZ 3109 commercialisée par la société UNION CARBIDE, de formule :

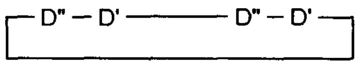

avec D" : 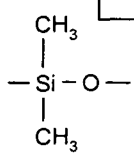 avec D' : 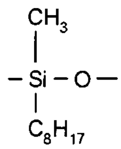

On peut également citer les mélanges de polydialkylsiloxanes cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bis-néopentane ;

Les polydialkylsiloxanes volatiles linéaires ayant 2 à 9 atomes de silicium et présentant une viscosité inférieure ou égale à 5.10-6m$^2$/s à 25°C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and Toiletries, Vol. 91, Jan. 76, P. 27-32 - TODD & BYERS "Volatile Silicone fluids for cosmetics".

On utilise de préférence des polydialkylsiloxanes non volatiles, des gommes et des résines de polydialkylsiloxanes, des polyorganosiloxanes modifiés par les groupements organofonctionnels ci-dessus ainsi que leurs mélanges.

Ces silicones sont plus particulièrement choisies parmi les polydialkylsiloxanes parmi lesquels on peut citer principalement les polydiméthylsiloxanes à groupements terminaux triméthylsilyl. La viscosité des silicones est mesurée à 25°C selon la norme ASTM 445 Appendice C.

Parmi ces polydialkylsiloxanes, on peut citer à titre non limitatif les produits commerciaux suivants :

- les huiles SILBIONE® des séries 47 et 70 047 ou les huiles MIRASIL® commercialisées par RHODIA telles que, par exemple l'huile 70 047 V 500 000 ;
- les huiles de la série MIRASIL® commercialisées par la société RHODIA ;
- les huiles de la série 200 de la société DOW CORNING telles que la DC200 ayant viscosité 60 000 mm2/s ;
- les huiles VISCASIL® de GENERAL ELECTRIC et certaines huiles des séries SF (SF 96, SF 18) de GENERAL ELECTRIC.

On peut également citer les polydiméthylsiloxanes à groupements terminaux diméthylsilanol connus sous le nom de dimethiconol (CTFA), tels que les huiles de la série 48 de la société RHODIA.

Dans cette classe de polydialkylsiloxanes, on peut également citer les produits commercialisés sous les dénominations "ABIL WAX® 9800 et 9801" par la société GOLDSCHMIDT qui sont des polydialkyl (C1-C20) siloxanes.

Les gommes de silicone utilisables conformément à l'invention sont notamment des polydialkylsiloxanes, de préférence des polydiméthylsiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles polyphénylméthylsiloxanes (PPMS), les isoparaffines, les polyisobutylènes, le chlorure de méthylène, le pentane, le dodécane, le tridécane ou leurs mélanges.

Des produits plus particulièrement utilisables conformément à l'invention sont des mélanges tels que :

- les mélanges formés à partir d'un polydiméthylsiloxane hydroxylé en bout de chaîne, ou diméthiconol (CTFA) et d'un polydiméthylsiloxane cyclique également appelé cyclométhicone (CTFA) tel que le produit Q2 1401 commercialisé par la société DOW CORNING ;
- les mélanges d'une gomme polydiméthylsiloxane et d'une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 $m^2/s$ et d'une huile SF 96 d'une viscosité de 5.10-6$m^2/s$. Ce produit comporte de préférence 15% de gomme SE 30 et 85% d'une huile SF 96.

Les résines d'organopolysiloxanes utilisables conformément à l'invention sont des systèmes siloxaniques réticulés renfermant les motifs :

$R_2SiO2/2$, $R_3SiO1/2$, RSiO3/2 et SiO4/2

dans lesquelles R représente un alkyl possédant 1 à 16 atomes de carbone. Parmi ces produits, ceux particulièrement préférés sont ceux dans lesquels R désigne un groupe alkyle inférieur en C1-C4, plus particulièrement méthyle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.

Outre, les silicones décrites ci-dessus les silicones organomodifiées peuvent être des polydiaryl siloxanes, notamment des polydiphénylsiloxanes, et des polyalkyl-arylsiloxanes fonctionnalisés par les groupes organofonctionnels mentionnés précédemment.

Les polyalkylarylsiloxanes sont particulièrement choisis parmi les polydiméthyl/méthylphénylsiloxanes, les polydiméthyl/diphénylsiloxanes linéaires et/ou ramifiés de viscosité allant de 1.10-5 à 5.10-2$m^2/s$ à 25°C.

Parmi ces polyalkylarylsiloxanes, on peut citer à titre d'exemple les produits commercialisés sous les dénominations suivantes :

- les huiles SILBIONE® de la série 70 641 de RHODIA;
- les huiles des séries RHODORSIL® 70 633 et 763 de RHODIA ;
- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000 ;
- certaines huiles des séries SF de GENERAL ELECTRIC telles que SF 1023, SF 1154, SF 1250, SF 1265.

Parmi les silicones organomodifiées, on peut citer les polyorganosiloxanes comportant :

- des groupements polyéthylèneoxy et/ou polypropylèneoxy comportant éventuellement des groupements alkyle en C6-C24 tels que les produits dénommés diméthicone copolyol commercialisé par la société DOW CORNING sous la dénomination DC 1248 ou les huiles SILWET® L 722, L 7500, L 77, L 711 de la société UNION CARBIDE et l'alkyl (C12)-méthicone copolyol commercialisée par la société DOW CORNING sous la dénomination Q2 5200 ;
- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en $C_1$-$C_4$ ;
- des groupements alcoxylés, comme le produit commercialisé sous la dénomination "SILICONE COPOLYMER F-755" par SWS SILICONES et ABIL WAX® 2428, 2434 et 2440 par la société GOLDSCHMIDT.

De préférence, les corps gras ne sont ni oxyalkylénés, ni glycérolés.

Plus particulièrement, les corps gras sont choisis parmi les composés liquides ou pâteux à température ambiante et à pression atmosphérique.

De préférence, le corps gras est un composé liquide à la température de 25°C et à la pression atmosphérique.

Les corps gras sont de préférence choisis parmi les alcanes inférieurs, les alcools gras, les esters d'acide gras, les esters d'alcool gras, les huiles en particulier les huiles non siliconées minérales, végétales ou synthétiques, les silicones

Selon un mode de réalisation, le ou les corps gras est ou sont choisis parmi l'huile de vaseline, les polydécènes, les esters d'acides gras ou d'alcools gras, liquides ou leurs mélanges, en particulier, le ou les corps gras de la composition selon l'invention sont non siliconés.

On choisira de préférence les alcanes ou hydrocarbures et les silicones.

La composition selon l'invention comprend au moins 25% de corps gras. De préférence la concentration en corps gras va de 25 à 80%, encore plus préférentiellement de 25 à 65%, mieux de 30 à 55% du poids total de la composition.

La composition selon l'invention comprend de préférence une quantité totale de N,N bis β-hydroxyéthyl paraphénylènediamine et/ou ses sels d'addition avec un acide variant de de 0,0005 à 12% en poids par rapport au poids total de la composition. De préférence, elle comprend une quantité totale de N,N bis β-hydroxyéthyl paraphénylènediamine et/ou ses sels d'addition avec un acide allant de 0,005 à 8% en poids, et mieux encore de 0,05 à 5 % en poids, par rapport au poids total de ladite composition.

En plus de la base d'oxydation choisie parmi la N,N bis β-hydroxyéthyl paraphénylènediamine et/ou ses sels d'addition avec un acide, la composition contient un ou plusieurs précurseurs de colorants additionnels.

Ce ou ces précurseurs de colorants additionnels sont choisis parmi les bases d'oxydation différentes de la N,N bis β -hydroxyéthyl paraphénylènediamine et/ou ses sels d'addition avec un acide, et les coupleurs.

La ou les bases d'oxydation utilisables dans le cadre de la présente invention sont choisies parmi celles classiquement connues en teinture d'oxydation, et parmi lesquelles on peut notamment citer les ortho- et para-phénylènediamines autres que la N,N bis β-hydroxyéthyl paraphénylènediamine et/ou ses sels d'addition avec un acide, les bases doubles, les ortho- et para- aminophénols, les bases hétérocycliques ainsi que les sels d'addition de ces composés avec un acide.

Ces bases d'oxydation peuvent être en particulier cationiques.

Les para-phénylènediamines utilisables dans le cadre de l'invention peuvent notamment être choisies parmi les composés de formule (II) suivante et leurs sels d'addition avec un acide :

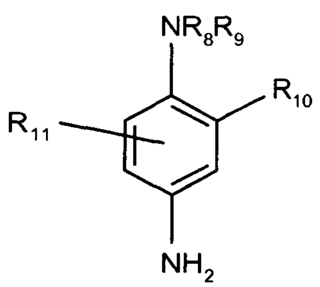

(II)

dans laquelle :

- ■ $R_8$ représente un atome d'hydrogène, un radical alkyle en $C_1$-$C_4$, monohydroxyalkyle en $C_1$-$C_4$, polyhydroxyalkyle en $C_2$-$C_4$, alcoxy($C_1$-$C_4$)alkyle($C_1$-$C_4$), alkyle en $C_1$-$C_4$ substitué par un groupement azoté, phényle ou 4'-aminophényle ;
- ■ $R_9$ représente un atome d'hydrogène, un radical alkyle en $C_1$-$C_4$, polyhydroxyalkyle en $C_2$-$C_4$, alcoxy($C_1$-$C_4$)alkyle($C_1$-$C_4$) ou alkyle en $C_1$-$C_4$ substitué par un groupement azoté ;
- ■ $R_8$ et $R_9$ peuvent également former avec l'atome d'azote qui les porte un hétérocycle azoté à 5 ou 6 chaînons éventuellement substitué par un ou plusieurs groupements alkyle, hydroxy ou uréido ;
- ■ $R_{10}$ représente un atome d'hydrogène, un atome d'halogène tel qu'un atome de chlore, un radical alkyle en $C_1$-$C_4$, sulfo, carboxy, monohydroxyalkyle en $C_1$-$C_4$ ou hydroxyalcoxy en $C_1$-$C_4$, acétylaminoalcoxy en $C_1$-$C_4$, mésylaminoalcoxy en $C_1$-$C_4$ ou carbamoylaminoalcoxy en $C_1$-$C_4$ ;
- ■ $R_{11}$ représente un atome d'hydrogène, d'halogène ou un radical alkyle en $C_1$-$C_4$.

Parmi les groupements azotés de la formule (II) ci-dessus, on peut citer notamment les radicaux amino, monoalkyl($C_1$-$C_4$)amino, dialkyl($C_1$-$C_4$)amino, trialkyl($C_1$-$C_4$)amino, monohydroxyalkyl($C_1$-$C_4$)amino, imidazolinium et am-

monium.

Parmi les para-phénylènediamines de formule (II) ci-dessus, on peut plus particulièrement citer la para-phénylènediamine, la para-toluylènediamine, la 2-chloro-para-phénylènediamine, la 2,3-diméthyl-para-phénylènediamine, la 2,6-diméthyl-para-phénylénediamine, la 2,6-diéthyl-para-phénylènediamine, la 2,5-diméthyl-para-phénylénediamine, la N,N-diméthyl-para-phénylénediamine, la N,N-diéthyl-para-phénylènediamine, la N,N-dipropyl-para-phénylènediamine, la 4-amino-N,N-diéthyl-3-méthyl-aniline, la 4-N,N-bis-(β-hydroxyéthyl)amino-2-méthyl-aniline, la 4-N,N-bis-(β-hydroxyéthyl)-amino-2-chloro-aniline, la 2-β-hydroxyéthyl-para- phénylènediamine, la 2-fluoro-para-phénylènediamine, la 2-isopropyl-para-phénylènediamine, la N-(β-hydroxypropyl)-para-phénylènediamine, la 2-hydroxyméthyl-para-phénylènediamine, la N,N-diméthyl-3-méthyl-para-phénylènediamine, la N,N-(éthyl,β-hydroxyéthyl)-para-phénylènediamine, la N-(β,γ-dihydroxypropyl)-para-phénylènediamine, la N-(4'-aminophényl)-para-phénylènediamine, la N-phényl-para-phénylènediamine, la 2-β-hydroxyéthyloxy-para-phénylènediamine, la 2-β-acétylaminoéthyloxy-para-phénylènediamine, la N-(β-méthoxyéthyl)-para-phénylènediamine, la 2-méthyl-1-N-β-hydroxyéthyl-para-phénylènediamine, et leurs sels d'addition avec un acide.

Parmi les para-phénylènediamines de formule (II) ci-dessus, on préfère tout particulièrement la para-phénylénediamine, la para-toluylènediamine, la 2-isopropyl-para-phénylènediamine, la 2-β-hydroxyéthyl-para-phénylènediamine, la 2,6-diméthyl-para-phénylènediamine, la 2,6-diéthyl-para-phénylènediamine, la 2,3-diméthyl-para-phénylènediamine, la 2-chloro-para-phénylènediamine, et leurs sels d'addition avec un acide.

On utilisera tout particulièrement la para-phénylènediamine et la para-toluylènediamine, et leurs sels d'addition avec un acide.

Selon l'invention, on entend par bases doubles les composés comportant au moins deux noyaux aromatiques sur lesquels sont portés des groupements amino et/ou hydroxyle.

Parmi les bases doubles utilisables à titre de bases d'oxydation dans la composition conforme à l'invention, on peut notamment citer les composés répondant à la formule (III) suivante et leurs sels d'addition avec un acide :

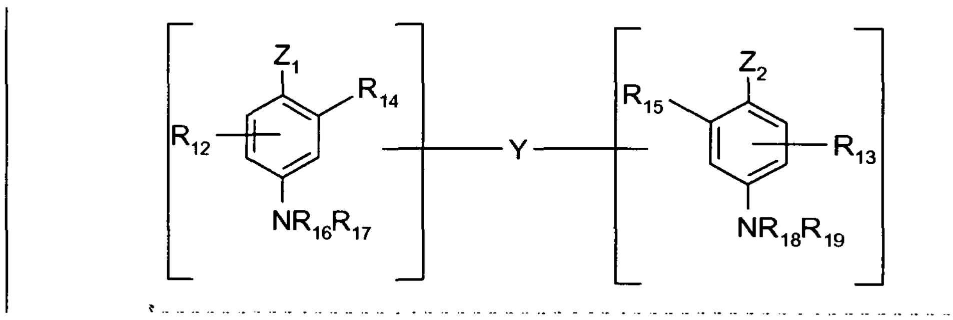

(III)

dans laquelle :

- $Z_1$ et $Z_2$, identiques ou différents, représentent un radical hydroxyle ou $-NH_2$ pouvant être substitué par un radical alkyle en $C_1$-$C_4$ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en $C_1$-$C_6$ ;
- $R_{12}$ et $R_{13}$ représentent un atome d'hydrogène ou d'halogène, un radical alkyle en $C_1$-$C_4$, monohydroxyalkyle en $C_1$-$C_4$, polyhydroxyalkyle en $C_2$-$C_4$, aminoalkyle en $C_1$-$C_4$ ou un bras de liaison Y ;
- $R_{14}$, $R_{15}$, $R_{16}$, $R_{17}$, $R_{18}$ et $R_{19}$, identiques ou différents, représentent un atome d'hydrogène, un bras de liaison Y ou un radical alkyle en $C_1$-$C_4$ ;
- étant entendu que les composés de formule (III) ne comportent qu'un seul bras de liaison Y par molécule.

Parmi les groupements azotés de la formule (III) ci-dessus, on peut citer notamment les radicaux amino, monoalkyl($C_1$-$C_4$)amino, dialkyl($C_1$-$C_4$)amino, trialkyl($C_1$-$C_4$)amino, monohydroxyalkyl($C_1$-$C_4$)amino, imidazolinium et ammonium.

Parmi les bases doubles de formules (III) ci-dessus, on peut plus particulièrement citer le N,N'-bis-(β-hydroxyéthyl)-N,N'-bis-(4'-aminophényl)-1,3-diamino-propanol, la N,N'-bis-(β-hydroxyéthyl)-N,N'-bis-(4'-aminophényl)-éthylènediamine, la N,N'-bis-(4-aminophényl)-tétraméthylènediamine, la N,N'-bis-(β-hydroxyéthyl)-N,N'-bis-(4-aminophényl)-tétraméthylènediamine, la N,N'-bis-(4-méthylaminophényl)-tétraméthylènediamine, la N,N'-bis-(éthyl)-N,N'-bis-(4'-amino-3'-méthylphényl)-éthylènediamine, le 1,8-bis-(2,5-diaminophénoxy)-3,5-dioxaoctane, et leurs sels d'addition avec

un acide.

Parmi ces bases doubles de formule (III), le N,N'-bis-(β-hydroxyéthyl)-N,N'-bis-(4'-aminophényl)-1,3-diamino-propanol, le 1,8-bis-(2,5-diaminophénoxy)-3,5-dioxaoctane ou l'un de leurs sels d'addition avec un acide sont particulièrement préférés.

Les para-aminophénols utilisables dans le cadre de l'invention peuvent notamment être choisis parmi les composés répondant à la formule (IV) suivante et leurs sels d'addition avec un acide :

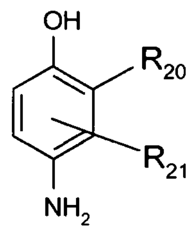

(IV)

dans laquelle :

- $R_{20}$ représente un atome d'hydrogène, un atome d'halogène tel que le fluor, un radical alkyle en $C_1$-$C_4$, monohydroxyalkyle en $C_1$-$C_4$, alcoxy($C_1$-$C_4$)alkyle($C_1$-$C_4$) ou aminoalkyle en $C_1$-$C_4$, ou hydroxyalkyl($C_1$-$C_4$)aminoalkyle en $C_1$-$C_4$ ;
- $R_{21}$ représente un atome d'hydrogène ou un atome d'halogène tel que le fluor, un radical alkyle en $C_1$-$C_4$, monohydroxyalkyle en $C_1$-$C_4$, polyhydroxyalkyle en $C_2$-$C_4$, aminoalkyle en $C_1$-$C_4$, cyanoalkyle en $C_1$-$C_4$ ou alcoxy($C_1$-$C_4$)alkyle($C_1$-$C_4$).

Parmi les para-aminophénols de formule (IV) ci-dessus, on peut plus particulièrement citer le para-aminophénol, le 4-amino-3-méthyl-phénol, le 4-amino-3-fluoro-phénol, le 4-amino-3-hydroxyméthyl-phénol, le 4-amino-2-méthyl-phénol, le 4-amino-2-hydroxyméthyl-phénol, le 4-amino-2-méthoxyméthyl-phénol, le 4-amino-2-aminométhyl-phénol, le 4-amino-2-(β-hydroxyéthyl-aminométhyl)-phénol, et leurs sels d'addition avec un acide.

Le para-aminophénol et le 4-amino-3-méthyl-phénol sont encore plus préférés.

Tout particulièrement on utilisera le 4-amino-3-méthyl-phénol.

Les ortho-aminophénols utilisables à titre de bases d'oxydation dans le cadre de la présente l'invention sont notamment choisis parmi le 2-amino-phénol, le 2-amino-1-hydroxy-5-méthyl-benzène, le 2-amino-1-hydroxy-6-méthyl-benzène, le 5-acétamido-2-amino-phénol, et leurs sels d'addition avec un acide.

Parmi les bases hétérocycliques utilisables à titre de bases d'oxydation dans la composition conforme à l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1026978 et GB 115 196, comme la 2,5-diamino-pyridine, la 2-(4-méthoxyphényl)amino-3-amino-pyridine, la 2,3-diamino-6-méthoxy-pyridine, la 2-(β-méthoxyéthyl)amino-3-amino-6-méthoxy pyridine, la 3,4-diamino-pyridine, et leurs sels d'addition avec un acide.

Parmi les dérivés pyrimidiniques, on peut plus particulièrement citer les composés décrits par exemple dans les brevets allemand DE 2359399 ou japonais JP 88-169571 et JP 91-10659 ou demandes de brevet WO 96/15765, comme la 2,4,5,6-tétra-aminopyrimidine, la 4-hydroxy-2,5,6-triaminopyrimidine, la 2-hydroxy-4,5,6-triaminopyrimidine, la 2,4-dihydroxy-5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl-pyrazolo-[1,5-a]-pyrimidine-3,7-diamine ; la pyrazolo-[1,5-a]-pyrimidine-3,5-diamine ; la 2,7-diméthyl-pyrazolo-[1,5-a]-pyrimidine-3,5-diamine ; le 3-amino-pyrazolo-[1,5-a]-pyrimidin-7-ol ; le 3-amino-pyrazolo-[1,5-a]-pyrimidin-5-ol ; le 2-(3-amino pyrazolo-[1,5-a]-pyrimidin-7-ylamino)-éthanol; le 2-(7-amino-pyrazolo-[1,5-a]-pyrimidin-3-ylamino)-éthanol ; le 2-[(3-amino-pyrazolo[1,5-a]pyrimidin-7-yl)-(2-hydroxy-éthyl)-amino]-éthanol ; le 2-[(7-amino-pyrazolo[1,5-a]pyrimidin-3-yl)-(2-hydroxy-éthyl)-amino]-éthanol; la 5,6-diméthyl-pyrazolo-[1,5-a]-pyrimidine-3,7-diamine ; la 2,6-diméthyl-pyrazolo-[1,5-a]-pyrimidine-3,7-diamine; la 2,5,N7,N7-tétraméthyl-pyrazolo-[1,5-a]-pyrimidine-3,7-diamine ; la 3-amino-5-méthyl-7-imidazolylpropylamino-pyrazolo-[1,5-a]-pyrimidine ; leurs sels d'addition et leurs formes tautomères, lorsqu'il existe un équilibre tautomérique.

Parmi les dérivés pyrazoliques, on peut plus particulièrement citer les composés décrits dans les brevets DE 3843892, DE 4133957 et demandes de brevet WO 94/08969, WO 94/08970, FR-A-2733749 et DE 19543988 comme les 4,5-diaminopyrazoles tels que par exemple le 4,5-diamino-1-méthyl-pyrazole, le 4,5-diamino-1-(4'-chlorobenzyl)-pyrazole, le 4,5-diamino-1,3-diméthyl-pyrazole, le 4,5-diamino-3-méthyl-1-phényl-pyrazole, le 4,5-diamino-1-méthyl-3-phényl-pyrazole, le 1-benzyl-4,5-diamino-3- méthyl-pyrazole, le 4,5-diamino-3-tert-butyl-1-méthyl-pyrazole, le 4,5-diamino-1-tert-butyl-3-méthyl-pyrazole, le 4,5-diamino-1-(β-hydroxyéthyl)-3-méthyl-pyrazole, le 4,5-diamino-1-(β-hydroxyéthyl)-pyrazole, le 4,5-diamino-1-éthyl-3-méthyl-pyrazole, le 4,5-diamino-1-éthyl-3-(4'-méthoxyphényl)-pyrazole, le 4,5-diamino-1-éthyl-3-hydroxyméthyl-pyrazole, le 4,5-diamine-3-hydroxyméthyl-1-méthyl-pyrazole, le 4,5-diamino-3-hydroxyméthyl-1-isopropyl-pyrazole, le 4,5-diamino-3-méthyl-1-isopropyl-pyrazole et le 4-amino-5-(2'-aminoéthyl)amino-1,3-diméthyl-pyrazole ; le 3,4-diamino-pyrazole ; le 4-amino-1,3-diméthyl-5-hydrazino-pyrazole ; les 3,4,5-triaminopyrazoles tels que par exemple le 3,4,5-triamino-pyrazole, le 1-méthyl-3,4,5-triamino-pyrazole, le 3,5-diamino-1-méthyl-4-méthylamino-pyrazole, le 3,5-diamino-4-(β-hydroxyéthyl)amino-1-méthyl-pyrazole ; et leurs sels d'addition avec un acide.

De préférence, on utilisera un 4,5-diaminopyrazole et encore plus préférentiellement le 4,5-diamino-1-(β-hydroxyéthyl)-pyrazole et/ou l'un des ses sels.

A titre de dérivés pyrazoliques, on peut également citer les diamino N,N-dihydropyrazolopyrazolones et notamment celles décrites dans la demande FR-A-2 886 136 telles que les composés suivants et leurs sels d'addition : 2,3-diamino-6,7-dihydro-1H,5H-pyrazolo[1,2-a]pyrazol-1-one, 2-amino-3-éthylamino-6,7-dihydro-1H,5H-pyrazolo[1,2-a]pyrazol-1-one, 2-amino-3-isopropylamino-6,7-dihydro-1H,5H-pyrazolo[1,2-a]pyrazol-1-one, 2-amino-3-(pyrrolidin-1-yl)-6,7-dihydro-1H,5H-pyrazolo[1,2-a]pyrazol-1-one, 4,5-diamino-1,2-diméthyl-1,2-dihydro-pyrazol-3-one, 4,5-diamino-1,2-diéthyl-1,2-dihydro-pyrazol-3-one, 4,5-diamino-1,2-di-(2-hydroxyéthyl)-1,2-dihydro-pyrazol-3-one, 2-amino-3-(2-hydroxyéthyl)amino-6,7-dihydro-1H,5H-pyrazolo[1,2-a]pyrazol-1-one, 2-amino-3-diméthylamino-6,7-dihydro-1H,5H-pyrazolo[1,2-a]pyrazol-1-one, 2,3-diamino-5,6,7,8-tétrahydro-1H,6H-pyridazino[1,2-a]pyrazol-1-one, 4-amino-1,2-diéthyl-5-(pyrrolidin-1-yl)-1,2-dihydro-pyrazol-3-one, 4-amino-5-(3-diméthylamino-pyrrolidin-1-yl)-1,2-diéthyl-1,2-dihydro-pyrazol-3-one, 2,3-diamino-6-hydroxy-6,7-dihydro-1H,5H-pyrazolo[1,2-a]pyrazol-1-one.

On préférera utiliser la 2,3-diamino-6,7-dihydro-1H,5H-pyrazolo[1,2-a]pyrazol-1-one et/ou un de ses sels d'addition.

A titre de bases hétérocycliques, on utilisera préférentiellement le 4,5-diamino-1-(β-hydroxyéthyl)pyrazole et/ou la 2,3-diamino-6,7-dihydro-1H,5H-pyrazolo[1,2-a]pyrazol-1-one et/ou un de leurs sels d'addition.

A titre de bases d'oxydation cationiques utilisables dans les compositions selon l'invention, on peut citer par exemple les composés suivants : les para-phénylènediamines telles que notamment décrites dans les demandes de brevets FR-A-2766177 et FR-A-2766178, les para-aminophénols tels que décrits par exemple dans les demandes de brevet FR-A-2766177 et FR-A-2766178, les ortho-phénylènediamines telles que décrites par exemple dans les demandes de brevet FR-A-2782718, FR-A-2782716 et FR-A-2782719, des ortho-aminophénols ou des bases doubles cationiques telles que des dérivés de type bis(aminophényl)alkylènediamine décrites dans les demandes de brevet FR-A-2766179, ainsi que les bases hétérocycliques cationiques, ces composés portant au moins un atome d'azote quaternaire.

De préférence, les bases d'oxydation cationiques utilisables dans les compositions selon l'invention sont des para-phénylènediamines cationiques.

De manière avantageuse, une variante consiste à mettre en oeuvre des bases d'oxydation cationiques de structure para-phénylènediamine, dont au moins une des fonctions aminé est une aminé tertiaire porteuse d'un noyau pyrrolidinique, la molécule possédant au moins un atome d'azote quaternisé. De telles bases sont, par exemple, décrites dans le document EP-A-1348695.

La composition selon l'invention comprend de préférence une quantité totale de bases d'oxydation allant de 0,0005 à 12% en poids par rapport au poids total de la composition. De préférence, elle comprend une quantité totale de bases d'oxydation allant de 0,005 à 8% en poids, et mieux encore de 0,05 à 5% en poids, par rapport au poids total de ladite composition.

Le ou les coupleurs utilisables dans la composition selon l'invention sont ceux classiquement utilisés dans les compositions de teinture d'oxydation, c'est-à-dire les méta-aminophénols, les méta-phénylènediamines, les métadiphénols, les naphtols et les coupleurs hétérocycliques tels que, par exemple, les dérivés indoliques, les dérivés indoliniques, le sésamol et ses dérivés, les dérivés pyridiniques, les dérivés pyrazolotriazoles, les pyrazolones, les indazoles, les benzimidazoles, les benzothiazoles, les benzoxazoles, les 1,3-benzodioxoles, les quinolines, et les sels d'addition de ces composés avec un acide.

Ces coupleurs sont plus particulièrement choisis parmi le 2,4-diamino 1-(β-hydroxyéthyloxy)-benzène, le 2-méthyl-5-amino-phénol, le 5-N-(β-hydroxyéthyl)amino-2-méthyl-phénol, le 3-amino-phénol, le 1,3-dihydroxy-benzène, le 1,3-dihydroxy-2-méthyl-benzène, le 4-chloro-1,3-dihydroxy-benzène, le 2-amino 4-(β-hydroxyéthylamino)-1-méthoxy-benzène, le 1,3-diamino-benzène, le 1,3-bis-(2,4-diaminophénoxy)-propane, le sésamol, le 1-amino-2-méthoxy-4,5-méthylènedioxy benzène, l'α-naphtol, le 6-hydroxy-indole, le 4-hydroxy-indole, le 4-hydroxy-N-méthyl indole, la 6-hydroxy-indoline, la 2,6-dihydroxy-4-méthyl-pyridine, le 1-H-3-méthyl-pyrazole-5-one, le 1-phényl-3-méthyl-pyrazole-5-one, la 2-amino-3-hydroxypyridine, le 3,6-diméthyl-pyrazolo-[3,2-c]-1,2,4-triazole, le 2,6-diméthyl-pyrazolo-[1,5-b]-1,2,4-triazole et leurs sels d'addition avec un acide.

Selon on mode de réalisation, la composition de l'invention comprend au moins un coupleur porteur d'au moins un groupe amino, éventuellement substitué, de préférence un coupleur aminophénol et/ou méta-phénylènediamine

Selon un mode de réalisation particulier, la composition comprend au moins un groupe NH2.

La composition selon l'invention comprend généralement une quantité totale de coupleurs allant de 0,0001 à 15% en poids par rapport au poids total de la composition. De préférence, elle comprend une quantité totale de coupleurs allant de 0,001 à 10% en poids, et mieux encore de 0,01 à 8% en poids, par rapport au poids total de la composition.

Les bases d'oxydation et coupleurs peuvent être présents dans les compositions de l'invention, sous forme de sels d'addition, et en particulier sous forme de sels d'addition avec un acide.

Les sels d'addition avec un acide utilisables dans le cadre de l'invention sont, notamment, choisis parmi les chlorhydrates, les bromhydrates, les sulfates, les citrates, les succinates, les tartrates, les lactates, les acétates, les alkylsulfates et les alkylsulfonates.

Lorsque les bases d'oxydation ou les coupleurs contiennent une ou plusieurs fonctions acide carboxylique ou sulfonique, des sels d'addition avec une base sont envisageables. Les sels d'addition avec une base utilisables dans le cadre des compositions tinctoriales de l'invention sont alors notamment ceux obtenus avec de la soude, de la potasse, de l'ammoniaque ou des aminés.

Selon un mode de réalisation particulier de l'invention, la composition comprend une ou plusieurs bases d'oxydation additionnelle et un ou plusieurs coupleurs.

Selon une variante, la base d'oxydation additionnelle est choisie parmi les para-aminophénols, les bases hétérocycliques ainsi que les sels d'addition avec un acide correspondant.

La composition conforme à la présente invention comprend un ou plusieurs agents oxydants.

Un tel agent oxydant est choisi par exemple parmi les peroxydes tels que le peroxyde d'hydrogène, le peroxyde d'urée, les bromates ou ferricyanures de métaux alcalins, les persels tels que les perborates, percarbonates et les persulfates. On peut également utiliser à titre d'agent oxydant une ou plusieurs enzymes d'oxydo-réduction telles que les laccases, les peroxydases et les oxydoréductases à 2 électrons (telles que l'uricase), éventuellement en présence de leur donneur ou cofacteur respectif.

L'utilisation du peroxyde d'hydrogène est particulièrement préférée. Cet agent oxydant est avantageusement constitué par une solution d'eau oxygénée dont le titre peut varier, plus particulièrement, d'environ 1 à 40 volumes, et encore plus préférentiellement d'environ 5 à 40 volumes.

La concentration en agents oxydants de la composition de l'invention va de préférence de 0.1 à 20% mieux de 0.5 à 10% du poids total de la composition.

La composition de l'invention peut comprendre un ou plusieurs agents alcalins. Ce ou ces agents alcalins sont par exemple choisis parmi l'ammoniaque, les carbonates ou bicarbonates alcalins et en particulier les carbonates ou bicarbonates de sodium ou de potassium, les alcanolamines telles que les mono-, di- et triéthanolamines ainsi que leurs dérivés, les hydroxyalkylamines et les éthylènediamines oxyéthylénées et/ou oxypropylénées, les hydroxydes de sodium ou de potassium, les acides aminés et en particulier les acides aminés basiques comme l'arginine ou la lysine et les composés de formule (V) suivante :

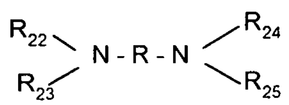

(V)

dans laquelle :

- R est un reste propylène éventuellement substitué par un groupement hydroxyle ou un radical alkyle en $C_1$-$C_4$ ;
- $R_{22}$, $R_{23}$, $R_{24}$ et $R_{25}$, identiques ou différents, représentent un atome d'hydrogène, un radical alkyle en $C_1$-$C_4$ ou hydroxyalkyle en $C_1$-$C_4$.

Selon un mode de réalisation particulier, la composition contient une faible quantité d'ammoniaque, voire pas d'ammoniaque. Selon ce mode de réalisation, la composition contient de préférence une ou plusieurs alcanolamines, notamment la monoéthanolamine ou le 2-amino 2-méthyl 1-propanol.

Selon un mode de réalisation particulier, la composition contient en tant qu'agents alcalins au moins une aminé organique, de préférence au moins une alkanolamine. Lorsque la composition contient plusieurs agents alcalins dont une alkanolamine et de l'ammoniaque ou un de ses sels, la ou les aminés organiques sont de préférence majoritaire en poids par rapport à la quantité ammoniac.

La concentration en agent(s) alcalin(s) de la composition de l'invention va de préférence de 0.01 à 30%, et encore plus préférentiellement de 0.1 à 20% du poids total de la composition.

La composition tinctoriale conforme à l'invention peut en outre contenir un ou plusieurs colorants directs pouvant notamment être choisis parmi les colorants nitrés de la série benzénique, les colorants directs azoïques, les colorants

directs méthiniques, et leurs sels d'addition. Ces colorants directs peuvent être de nature non-ionique, anionique ou cationique.

La composition peut aussi contenir d'autres composés constituant le milieu de coloration. Ce milieu de coloration comprend généralement de l'eau ou un mélange d'eau et d'un ou plusieurs solvant(s) organique(s) acceptable(s), de préférence hydrosolubles sur le plan cosmétique.

A titre d'exemples de solvants organiques, on peut notamment citer les alcools tels que l'alcool éthylique, l'alcool isopropylique, l'alcool benzylique, et l'alcool phényléthylique, ou les glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, l'hexylène glycol, le dipropylèneglycol ainsi que les alkyléthers de diéthylèneglycol comme par exemple, le monoéthyléther ou le monobutyléther du diéthylèneglycol. Les solvants peuvent alors être présents dans des concentrations comprises entre environ 0,01 à 35% en poids et, de préférence, entre environ 0,1 et 25% en poids par rapport au poids total de la composition.

De préférence la composition de l'invention contient de l'eau. Encore plus préférentiellement la concentration en eau peut aller de 10 à 70%, mieux de 20 à 55% du poids total de la composition.

La composition conforme à l'invention peut contenir en outre un ou plusieurs adjuvant(s) utilisé(s) classiquement dans les compositions pour la teinture des cheveux.

Par "adjuvant", on entend un additif, différent des composés précités.

A titre d'exemples d'adjuvants utilisables, on peut citer les agents tensio-actifs anioniques, cationiques, non-ioniques, amphotères, zwittérioniques ou leurs mélanges ; les polymères anioniques, cationiques, non-ioniques, amphotères, zwittérioniques, les agents épaississants minéraux ou organiques, et en particulier les épaississants associatifs polymériques anioniques, cationiques, non- ioniques et amphotères, autres que les celluloses associatives selon l'invention ; les agents antioxydants ou réducteurs ; les agents de pénétration ; les agents séquestrants ; les parfums ; les tampons ; les agents dispersants ; les agents conditionneurs tels que par exemple les silicones volatiles ou non volatiles, modifiées ou non modifiées ; les agents filmogènes ; les céramides ; les agents conservateurs ; les agents opacifiants ; et les agents anti-statique.

De préférence la composition de l'invention contient un ou plusieurs tensioactifs.

De préférence, le ou les tensioactifs sont choisis parmi les tensioactifs non ioniques ou parmi les tensioactifs anioniques.

Les tensioactifs anioniques sont plus spécialement choisis parmi les sels (en particulier sels de métaux alcalins, notamment de sodium, sels d'ammonium, sels d'amines tels que les sels d'aminoalcools ou sels de métaux alcalino-terreux comme le magnésium) des composés suivants :

- les alkylsulfates, les alkyléthersulfates, alkylamidoéthersulfates, alkylaryl-polyéthersulfates, monoglycérides sulfates ;
- les alkylsulfonates, alkylamidesulfonates, alkylarylsulfonates, $\alpha$ - oléfine-sulfonates, paraffine-sulfonates ;
- les alkylphosphates, les alkylétherphosphates;
- les alkylsulfosuccinates, les alkyléthersulfosuccinates, les alkylamide-sulfosuccinates; les alkylsulfosuccinamates ;
- les alkylsulfoacétates ;
- les acylsarcosinates ; les acyliséthionates et les N-acyltaurates ;
- les sels d'acides gras tels que les acides oléique, ricinoléique, palmitique, stéarique, les acides d'huile de coprah ou d'huile de coprah hydrogénée ;
- les sels d'acides d'alkyl D galactoside uroniques ;
- les acyl-lactylates ;
- les sels des acides alkyléther carboxyliques polyoxyalkylénés, des acides alkylaryléther carboxyliques polyoxyalkylénés, des acides alkylamidoéther carboxyliques polyoxyalkylénés, en particulier ceux comportant de 2 à 50 groupements oxyde d'éthylène ;
- et leurs mélanges.

Il est à noter que le radical alkyle ou acyle de ces différents composés comporte avantageusement de 6 à 24 atomes de carbone, et de préférence de 8 à 24 atomes de carbone, et le radical aryle désignant de préférence un groupement phényle ou benzyle.

Les tensioactifs non ioniques sont plus particulièrement choisis parmi les tensioactifs non ioniques mono ou poly- oxyalkylénés, mono- ou poly- glycérolés. Les motifs oxyalkylénés sont plus particulièrement des motifs oxyéthylénés, oxypropylénés, ou leur combinaison, de préférence oxyéthylénés.

A titre d'exemples de tensioactifs non ioniques oxyalkylénés, on peut citer :

- les alkyl($C_8$-$C_{24}$)phénols oxyalkylénés,
- les alcools en $C_8$-$C_{30}$, saturés ou non, linéaires ou ramifiés, oxyalkylénés,

- les amides, en $C_8$-$C_{30}$, saturés ou non, linéaires ou ramifiés, oxyalkylénés,
- les esters d'acides en $C_8$-$C_{30}$, saturés ou non, linéaires ou ramifiés, et de polyéthylèneglycols,
- les esters d'acides en $C_8$-$C_{30}$, saturés ou non, linéaires ou ramifiés, et de sorbitol polyoxyéthylénés,
- les huiles végétales oxyéthylénées, saturées ou non,
- les condensats d'oxyde d'éthylène et/ou d'oxyde de propylène, entre autres, seuls ou en mélanges.

Les tensioactifs présentant un nombre de moles d'oxyde d'éthylène et/ou de propylène compris entre 1 et 50, de préférence entre 2 et 30. De manière avantageuse, les tensioactifs non ioniques ne comprennent pas de motifs oxypropylénés.

Conformément à un mode de réalisation préféré de l'invention, les tensioactifs non ioniques oxyalkylénés sont choisis parmi les alcools en $C_8$-$C_{30}$, oxyéthylénés, de préference $C_{18}$-$C_{30}$, oxyéthylénés.

A titre d'exemple de tensioactifs non ioniques mono- ou poly-glycérolés, on utilise de préference les alcools en $C_8$-$C_{40}$, mono- ou poly- glycérolés.

En particulier, les alcools en $C_8$-$C_{40}$ mono- ou poly- glycérolés correspondent à la formule suivante :

$$RO-[CH_2-CH(CH_2OH)-O]_m-H$$

dans laquelle R représente un radical alkyle ou alcényle, linéaire ou ramifié, en $C_8$-$C_{40}$, de préference en $C_8$-$C_{30}$, et m représente un nombre allant de 1 à 30 et de préférence de 1 à 10.

A titre d'exemple de composés convenables dans le cadre de l'invention, on peut citer, l'alcool laurique à 4 moles de glycérol (nom INCI : POLYGLYCERYL-4 LAURYL ETHER), l'alcool laurique à 1,5 moles de glycérol, l'alcool oléique à 4 moles de glycérol (nom INCI : POLYGLYCERYL-4 OLEYL ETHER), l'alcool oléique à 2 moles de glycérol (Nom INCI : POLYGLYCERYL-2 OLEYL ETHER), l'alcool cétéarylique à 2 moles de glycérol, l'alcool cétéarylique à 6 moles de glycérol, l'alcool oléocétylique à 6 moles de glycérol, et l'octadécanol à 6 moles de glycérol.

L'alcool peut représenter un mélange d'alcools au même titre que la valeur de m représente une valeur statistique, ce qui signifie que dans un produit commercial peuvent coexister plusieurs espèces d'alcools gras polyglycérolés sous forme d'un mélange.

Parmi les alcools mono- ou poly-glycérolés, on préfère plus particulièrement utiliser l'alcool en $C_8/C_{10}$ à une mole de glycérol, l'alcool en $C_{10}/C_{12}$ à 1 mole de glycérol et l'alcool en $C_{12}$ à 1,5 mole de glycérol.

De préférence, le tensioactif présent dans la composition de l'invention est un tensioactif non ionique.

La teneur en tensioactifs dans la composition de l'invention représente plus particulièrement de 0,1 à 50% en poids, de préférence de 0,5 à 30% en poids par rapport au poids de la composition.

Les adjuvants ci-dessus sont en général présents en quantité comprise pour chacun d'eux entre 0,01 et 20% en poids par rapport au poids de la composition tinctoriale.

Bien entendu, l'homme de l'art veillera à choisir le(s) éventuel(s) adjuvant(s) mentionné(s) ci-avant(s), de manière telle que les propriétés avantageuses attachées intrinsèquement aux compositions selon l'invention ne soient pas, ou substantiellement pas, altérées par l' adjonction (les adjonctions) envisagée(s).

Le pH de la composition conforme à l'invention est généralement compris entre 3 et 12 environ, de préférence entre 5 et 11 environ, préférentiellement 7 à 11. Il peut être ajusté à la valeur désirée au moyen d'agents acidifiants ou alcalinisants habituellement utilisés en teinture des fibres kératiniques ou bien encore à l'aide de systèmes tampons classiques.

Les agents alcalins sont par exemple ceux décrits précédemment.

Parmi les agents acidifiants, on peut citer, à titre d'exemple, des acides minéraux ou organiques comme l'acide chlorhydrique, l'acide ortho-phosphorique, des acides carboxyliques comme l'acide tartrique, l'acide citrique, l'acide lactique, ou des acides sulfoniques.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Le procédé de la présente invention est un procédé dans lequel on applique sur les fibres la composition selon la présente invention telle que définie précédemment. La couleur peut être révélée à pH acide, neutre ou alcalin et l'agent oxydant peut être ajouté juste au moment de l'emploi ou il peut être mis en oeuvre simultanément ou séquentiellement aux autres composés de la composition de l'invention. De préférence, cette coloration est révélée à pH neutre.

Après un temps de pose variant généralement de 1 à 60 minutes environ, de préférence 5 à 45 minutes environ, les fibres kératiniques sont rincées, éventuellement lavées au shampooing et rincées à nouveau, puis séchées.

La composition selon l'invention peut résulter du mélange d'au moins deux compositions et de préférence de 2 ou 3 compositions dont préférentiellement une composition oxydante comprenant au moins un agent oxydant tel que défini précédemment.

L'invention a aussi pour objet un dispositif à plusieurs compartiments ou "kit" de teinture dans lequel un premier

compartiment contient une composition comprenant le ou les corps gras, un second compartiment comprenant la ou les N,N bis β-hydroxyéthyl para phénylènediamine et le ou les précurseurs de colorant et l'agent alcalin et un troisième compartiment comprenant l'agent oxydant, ce troisième compartiment pouvant contenir tout ou partie des corps gras. Dans ce mode de réalisation, la composition comprenant le ou les corps gras peut être anhydre. On entend, par composition anhydre, au sens de l'invention, une composition cosmétique présentant une teneur en eau inférieure à 5 % en poids, de préférence inférieure à 2% en poids et de manière encore plus préférée inférieure à 1% en poids par rapport au poids de ladite composition. Il est à noter qu'il s'agit plus particulièrement d'eau liée, comme l'eau de cristallisation des sels ou des traces d'eau absorbée par les matières premières utilisées dans la réalisation des compositions selon l'invention.

Selon un second mode de réalisation, le dispositif de l'invention comprend un premier compartiment contenant une composition comprenant le ou les corps gras et un ou plusieurs agents oxydants et un second compartiment contenant une composition comprenant la ou les N,N bis β-hydroxyéthyl para phénylènediamine, le ou les précurseurs de colorant additionnels et un ou plusieurs agents alcalins. Ce dispositif peut être équipé d'un moyen permettant de délivrer sur les cheveux le mélange souhaité, tel que les dispositifs décrits dans le brevet FR-A-2 586 913 au nom de la Demanderesse.

Selon un troisième mode de réalisation, le dispositif de l'invention comprend un premier compartiment contenant une composition comprenant le ou les corps gras, la ou les N,N-bis β-hydroxyéthyl para phénylènediamine, le ou les colorants additionnels, le ou les agents alcalins et un second compartiments contenant un ou plusieurs agents oxydants.

La présente invention a également pour objet l'utilisation pour la teinture d'oxydation des fibres kératiniques, et en particulier des fibres kératiniques humaines telles que les cheveux, d'une composition tinctoriale telle que définie précédemment.

Les exemples qui suivent sont destinés à illustrer l'invention sans pour autant en limiter la portée.

Dans ces exemples, toutes les quantités sont indiquées en pourcent en poids de matière active (M.A.) par rapport au poids total de la composition, sauf indication contraire.

**EXEMPLE**

Les compositions suivantes ont été préparées :

| **Composition 1** | **Concentration (g%)** |
|---|---|
| DISTEARDIMONIUM HECTORITE | 3 |
| OCTYLDODECANOL | 11.5 |
| GLYCOL DISTEARATE | 8 |
| HUILE DE VASELINE | 64.5 |
| PROPYLENE CARBONATE | 1 |
| LAURETH-2 | 1 |
| POLYSORBATE 21 | 11 |

| **Composition 2** | **concentration (g%)** |
|---|---|
| séquestrant | 1 |
| Métabisulfite de sodium | 0.7 |
| monoethanolamine | 14.5 |
| 1-METHYL-2,5-DIAMINO-BENZENE | 7.25 |
| SULFATE DE N,N-BIS(2-HYDROXYETHYL)-P-PHENYLENEDIAMINE, 1 H2O | 0.58 |
| 1,3-DIHYDROXYBENZENE | 5.8 |
| 1-HYDROXY-3-AMINO-BENZENE | 1.45 |
| 1-BETA-HYDROXYETHYLOXY-2,4-DIAMINO-BENZENE DICHLORHYDRATE | 0.58 |
| NATROSOL 250 HHR (hydroxyethylcellulose) | 1.5 |

(suite)

| Composition 2 | concentration (g%) |
|---|---|
| HEXYLENE GLYCOL | 3 |
| DIPROPYLENE GLYCOL | 3 |
| ALCOOL ETHYLIQUE | 8.25 |
| propyleneglycol | 6.2 |
| Acide ascorbique | 0.25 |
| eau | Qs 100 g |

| Composition 3 | Concentration (g%) |
|---|---|
| PENTASODIUM PENTETATE | 0.15 |
| PEROXYDE D'HYDROGENE EN SOLUTION A 50 % (EAU OXYGENEE 200 VOL.) | 12 |
| SODIUM STANNATE | 0.04 |
| TETRASODIUM PYROPHOSPHATE | 0.03 |
| HUILE DE VASELINE | 20 |
| HEXADIMETHRINE CHLORIDE (MA à 60% ans l'eau) | 0.25 |
| POLYQUATERNIUM-6 ( MA à 40% dans l'eau) | 0.5 |
| eau | 54.1 |
| glycerine | 0.5 |
| ALCOOL CETYLSTEARYLIQUE (C16/C18 30/70) | 8 |
| ALCOOL CETYLSTEARYLIQUE OXYETHYLENE (33 OE) | 3 |
| AMIDE D'ACIDES DE COLZA OXYETHYLENE (4 OE) PROTEGE à 92.3 % dans l'eau | 1.3 |
| VITAMINE E | 0,1 |
| ACIDE PHOSPHORIQUE | Qs pH 2.2 |

Les trois compositions sont mélangées au moment de l'emploi dans les proportions suivantes : 10 g de la composition 1 avec 4 g de la composition 2 et 16 g de la composition 3. Le mélange est appliqué sur des mèches de cheveux gris naturels à 90% de cheveux blancs à raison de 10 g de mélange pour 1 g de cheveux. Après 30 min de pause, les cheveux sont rincés, lavés avec un shampooing standard et séchés.

La coloration capillaire est évaluée de manière visuelle.

| **Exemple 1** | Noir Intense |
|---|---|

**Exemple 2**

Les compositions suivantes ont été préparées (quantité exprimée en g)

| | A3 | A4 (invention) |
|---|---|---|
| Myristate d'isopropyle | 52 | 87 |
| Oleth-10 | 10 | 10 |
| Disteardimonium hectorite | 2,25 | 2,25 |
| Carbonate de propylène | 0,75 | 0,75 |

(suite)

| | A3 | A4 (invention) |
|---|---|---|
| Eau | 35 | - |

Composition B' (en g)

| | |
|---|---|
| N,N-bis(2-hydroxyéthyl) p-phénylènediamine sulfate hydrate | 4,524 |
| 6-hydroxy benzomorpholine | 2,1895 |
| Hydroxyéthyl cellulose (Natrosol 250 HHR) | 1,5 |
| Dipropylène glycol | 3 |
| Hexylène glycol | 3 |
| Propylène glycol | 6,2 |
| Monoéthanolamine | 15,92 |
| Alcool éthylique | 8,25 |
| Réducteurs, séquestrant | qs |
| Eau | Qs 100 |

Composition C (en g)

| | |
|---|---|
| Peroxyde d'hydrogène | 6 |
| Alcool cétéarylique | 2,28 |
| Ceteareth-25 | 0,57 |
| Trideceth-2 carboxamide MEA | 0,85 |
| Glycérine | 0,5 |
| Stabilisants, séquestrants | Qs |
| Acide phosphorique | Qs pH = 2 |
| Eau | Qs 100 |

Au moment de l'emploi, on mélange 10g de composition A3 ou A4 avec 4 g de composition B' et 15 g de composition C.

Chaque mélange est ensuite appliqué sur des mèches de cheveux à 90% blancs naturels (BN) et permanentés (BP). Après 30 minutes de pause à température ambiante, les cheveux sont rincés, lavés avec un shampooing standard et séchés.

Les mesures colorimétriques sont réalisées à l'aide d'un spectrocolorimètre Datacolor SF600X Spectraflash (illuminant D65, angle 10°, composantes spéculaires incluses). La coloration des cheveux est évaluée dans le système L*a*b* system,

Dans ce système, L représente l'intensité, plus la valeur de L* est faible, plus la coloration obtenue est intense La chromaticité est mesurée par les valeurs a* et b*, a* représentant l'axe rouge/vert et b* l'axe jaune/bleu

**<u>Sélectivité :</u>**

La sélectivité de la coloration obtenue à partir de ces compositions a été évaluée. La sélectivité de la coloration est la variation de la couleur entre des cheveux différemment sensibilisés. La sélectivité est représentative de l'uniformité de la coloration le long d'une mèche de cheveux puisque les cheveux ne sont en général pas sensibilisés de la même façon entre la racine et la pointe.

La sélectivité est mesurée par :

$\Delta$ E, qui est la variation de la couleur entre les différents types de cheveux est obtenu à partir de la formule :

$$\Delta E = \sqrt{(L^* - L_o^*)^2 + (a^* - a_o^*)^2 + (b^* - b_o^*)^2}$$

Dans laquelle L* représente l'intensité a* et b*, la chromaticité des cheveux colorés permanentés et L0* représente l'intensité et a0* et b0* la chromaticité des cheveux colorés naturels. Plus la valeur de ΔE est faible, plus la sélectivité est faible et la coloration uniforme le long des cheveux.

| Mélange | Type cheveux | L* | a* | b* | ΔE |
|---|---|---|---|---|---|
| A3 + B' + C | BN | 29,7 | -0,6 | 4,8 | 11,1 |
| | BP | 19,2 | -0,4 | 1,3 | |
| A4 + B' + C (invention) | BN | 28,9 | -0,6 | 3,8 | 7,7 |
| | BP | 21,4 | -0,6 | 2,3 | |

Le mélange obtenu avec la composition A4 conduit une sélectivité plus faible, donc une meilleure homogénéité de la coloration, par rapport au mélange obtenu avec la composition A3.

**Revendications**

**1.** Composition pour la teinture d'oxydation des fibres kératiniques, comprenant,

A) au moins 25 % de corps gras différents des acides gras,
B) une ou plusieurs bases d'oxydation choisies parmi la N,N bis β-hydroxyéthyl paraphénylène diamine et les sels d'addition avec un acide correspondants,
C) un ou plusieurs précurseurs de colorants additionnels différents de la base d'oxydation définie sous B),
D) un ou plusieurs agents oxydants, et éventuellement
E) un ou plusieurs agents alcalins

**2.** Composition selon la revendication 1 **caractérisé en ce que** le ou les corps gras sont choisis parmi les composés liquides ou pâteux à température ambiante et à pression atmosphérique.

**3.** Composition selon l'une quelconque des revendications précédentes dans laquelle le ou les corps gras sont choisis parmi les alcanes, les alcools gras, les esters d'acides gras, les esters d'alcool gras, les huiles et les cires.

**4.** Composition selon l'une quelconque des revendications précédentes dans laquelle le corps gras est non siliconé.

**5.** Composition selon l'une quelconque des revendications précédentes, **caractérisée en ce que** le précurseur de colorant additionnel est choisi parmi les bases d'oxydation et les coupleurs.

**6.** Composition selon la revendication précédente, **caractérisée en ce que** le précurseur de colorant est choisi parmi les bases d'oxydation ortho- et para-phénylènediamines, les bases doubles, les ortho- et para-aminophénols, les bases hétérocycliques, ainsi que les sels d'addition de ces composés avec un acide.

**7.** Composition selon la revendication précédente, **caractérisée en ce que** le précurseur de colorants est choisi parmi les bases d'oxydation para-aminophénols, les bases hétérocycliques, ainsi que les sels d'addition de ces composés avec un acide.

**8.** Composition selon la revendication 5, **caractérisée en ce que** le précurseur de colorant est choisi parmi les coupleurs méta-aminophénols, les méta-phénylènediamines, les méta-diphénols, les naphtols, les coupleurs hétérocycliques et les sels d'addition de ces composés avec un acide, de préférence un coupleur aminophénol et/ou méta-phénylènediamine.

**9.** Composition selon l'une quelconque des revendications précédentes, **caractérisée en ce que** l'agent oxydant est un peroxyde, de préférence le peroxyde d'hydrogène.

**10.** Composition selon l'une quelconque des revendications précédentes dans laquelle l'agent alcalin est l'ammoniac ou une alcanolamine, de préférence une alcanolamine.

**11.** Procédé de teinture des fibres kératiniques, **caractérisé en ce qu'**une composition telle que définie à l'une quelconque des revendications 1 à 10 est appliquée sur les fibres kératiniques pendant une durée suffisante pour développer la coloration désirée.

**12.** Dispositif à plusieurs compartiments comprenant un premier compartiment contient une composition comprenant le ou les corps gras différents des acides gras, un second compartiment comprenant la ou les N,N bis β-hydroxyéthyl para phénylènediamine et le ou les précurseurs de colorant et l'agent alcalin et un troisième compartiment comprenant l'agent oxydant, ce troisième compartiment pouvant contenir des corps gras ; le corps gras, les précurseurs de colorant, l'agent oxydant et l'agent alcalin étant définis selon l'une quelconque des revendications précédentes.

**13.** Dispositif à plusieurs compartiments comprenant un premier compartiment qui contient une composition comprenant le ou les corps gras différents des acides gras et un ou plusieurs agents oxydants et un second compartiment comprenant la ou les N,N bis β-hydroxyéthyl para phénylènediamine, le ou les précurseurs de colorant et l'agent alcalin, le corps gras, le précurseur de colorant, l'agent oxydant et l'agent alcalin étant définis selon l'une quelconque des revendications précédentes.

**14.** Dispositif à plusieurs compartiments comprenant un premier compartiment contenant une composition comprenant le ou les corps gras différents des acides gras, la ou les N,N-bis β-hydroxyéthyl para phénylènediamine, le ou les colorants additionnels, le ou les agents alcalins et un second compartiments contenant un ou plusieurs agents oxydants, le corps gras, le précurseur de colorant, l'agent oxydant et l'agent alcalin étant définis selon l'une quelconque des revendications précédentes.

**15.** Utilisation pour la teinture d'oxydation des fibres kératiniques d'une composition telle que définie à l'une quelconque des revendications 1 à 10.

## Patentansprüche

**1.** Zusammensetzung zum Oxidationsfärben von Keratinfasern, umfassend

A) mindestens 25% Fettsubstanzen, die von Fettsäuren verschieden sind,
B) eine oder mehrere Oxidationsbasen, ausgewählt aus N,N-Bis-β-hydroxyethyl-para-phenylendiamin und den entsprechenden Säureadditionssalzen,
C) eine oder mehrere zusätzliche Farbstoffvorstufen, die von der unter B) definierten Oxidationsbase verschieden sind,
D) ein oder mehrere Oxidationsmittel und gegebenenfalls
E) ein oder mehrere alkalische Mittel.

**2.** Zusammensetzung nach Anspruch 1, **dadurch gekennzeichnet, dass** die Fettsubstanz bzw. die Fettsubstanzen aus Verbindungen, die bei Umgebungstemperatur und Normaldruck flüssig oder pastös sind, ausgewählt ist bzw. sind.

**3.** Zusammensetzung nach einem der vorhergehenden Ansprüche, wobei die Fettsubstanz bzw. die Fettsubstanzen unter Alkanen, Fettalkoholen, Fettsäureestern, Fettalkoholestern, Ölen und Wachsen ausgewählt ist bzw. sind.

**4.** Zusammensetzung nach einem der vorhergehenden Ansprüche, wobei die Fettsubstanz nicht auf Silikon basiert.

**5.** Zusammensetzung nach einem der vorhergehenden Ansprüche, **dadurch gekennzeichnet, dass** die zusätzliche Farbstoffvorstufe aus Oxidationsbasen und Kupplern ausgewählt ist.

**6.** Zusammensetzung nach dem vorhergehenden Anspruch, **dadurch gekennzeichnet, dass** die Farbstoffvorstufe aus ortho- und para-Phenylendiamin-Oxidationsbasen, Doppelbasen, ortho- und para-Aminophenole, heterocyclischen Basen sowie Säureadditionssalzen dieser Verbindungen ausgewählt ist.

**7.** Zusammensetzung nach dem vorhergehenden Anspruch, **dadurch gekennzeichnet, dass** die Farbstoffvorstufe

unter para-Aminophenol-Oxidationsbasen, heterocyclischen Basen sowie Säureadditionssalzen dieser Verbindungen ausgewählt ist.

8. Zusammensetzung nach Anspruch 5, **dadurch gekennzeichnet, dass** die Farbstoffvorstufe aus meta-Aminophenol-, meta-Phenylendiamin-, meta-Diphenol- und Naphthol-Kupplern, heterocyclischen Kupplern und Säureadditionssalzen dieser Verbindungen, vorzugsweise einem Aminophenol- und/oder meta-Phenylendzamin-Kuppler, ausgewählt ist.

9. Zusammensetzung nach einem der vorhergehenden Ansprüche, **dadurch gekennzeichnet, dass** es sich bei dem Oxidationsmittel um ein Peroxid, vorzugsweise Wasserstoffperoxid, handelt.

10. Zusammensetzung nach einem der vorhergehenden Ansprüche, wobei es sich bei dem alkalischen Mittel um Ammoniak oder ein Alkanolamin, vorzugsweise ein Alkanolamin, handelt.

11. Verfahren zum Färben von Keratinfasern, **dadurch gekennzeichnet, dass** man auf die Keratinfasern eine Zusammensetzung gemäß einem der Ansprüche 1 bis 10 über eignen zur Entwicklung der gewünschten Färbung ausreichenden Zeitraum aufbringt.

12. Vorrichtung mit mehreren Kompartimenten mit einem ersten Kompartiment, das eine Zusammensetzung, die die Fettsubstanz bzw. die Fettsubstanzen, die von Fettsäuren verschieden ist bzw. sind, umfasst, enthält, einem zweiten Kompartiment, das das N,N-Bis-β-hydroxyethyl-para-phenylendiamin bzw. die N,N-Bis-β-hydroxyethyl-para-phenylendiamine und die Farbstoffvorstufe bzw. die Farbstoffvorstufen und das alkalische Mittel umfasst, und einem dritten Kompartiment, das das Oxidationsmittel umfasst, wobei dieses dritte Kompartiment Fettsubstanzen enthalten kann; wobei die Fettsubstanz, die Farbstoffvorstufen, das Oxidationsmittel und das alkalische Mittel gemäß einem der vorhergehenden Ansprüche definiert sind.

13. Vorrichtung mit mehreren Kompartimenten mit einem ersten Kompartiment, das eine Zusammensetzung, die die Fettsubstanz bzw. die Fettsubstanzen, die von Fettsäuren verschieden ist bzw. sind, und ein oder mehrere Oxidationsmittel umfasst, enthält, und einem zweiten Kompartiment, das das N,N-Bis-β-hydryxyethyl-para-phenylendiamin bzw. die N,N-Bis-β-hydroxyethyl-para-phenylendiamine, die Farbstoffvorstufe bzw. die Farbstoffvorstufen, das Oxidationsmittel und das alkalische Mittel, umfasst, wobei die Fettsubstanz, die Farbstoffvorstufe, das Oxidationsmittel und das alkalische Mittel gemäß einem der vorhergehenden Ansprüche definiert sind.

14. Vorrichtung mit mehreren Kompartimenten mit einem ersten Kompartiment, das eine Zusammensetzung, die die Fettsubstanz bzw. die Fettsubstanzen, die von Fettsäuren verschieden ist bzw. sind, das N,N-Bis-β-hydroxyethyl-para-phenylendiamin bzw. die N,N-Bis-β-hydroxyethyl-para-phenylendiamine, der zusätzliche Farbstoff bzw. die zusätzlichen Farbstoffe und das bzw. die alkalische(n) Mittel umfasst, enthält, und einem zweiten Kompartiment, das ein oder mehrere Oxidationsmittel umfasst, wobei die Fettsubstanz, die Farbstoffvorstufe, das Oxidationsmittel und das alkalische Mittel gemäß einem der vorhergehenden Ansprüche definiert sind.

15. Verwendung einer Zusammensetzung gemäß einem der Ansprüche 1 bis 10 zum Oxidationsfärben von Keratinfasern.

## Claims

1. Composition for the oxidation dyeing of keratin fibers, comprising:

   A) at least 25% of fatty substances other than fatty acids,
   B) one or more oxidation bases chosen from N,N-bis(β-hydroxyethyl)-para-phenylenediamine and the corresponding addition salts with an acid,
   C) one or more additional dye precursors different than the oxidation base defined in B),
   D) one or more oxidizing agents, and optionally
   E) one or more alkaline agents.

2. Composition according to Claim 1, **characterized in that** the fatty substance(s) is (are) chosen from compounds which are liquid or pasty at ambient temperature and at atmospheric pressure.

3. Composition according to either one of the preceding claims, **characterized in that** the fatty substance(s) is (are) chosen from alkanes, fatty alcohols, fatty acid esters, fatty alcohol esters, oils and waxes.

4. Composition according to any one of the preceding claims, in which the fatty substance is non-silicone-based.

5. Composition according to any one of the preceding claims, **characterized in that** the additional dye precursor is chosen from oxidation bases and couplers.

6. Composition according to the preceding claim, **characterized in that** the dye precursor is chosen from ortho- and para-phenylenediamine oxidation bases, double bases, ortho- and para-aminophenols, heterocyclic bases and also the addition salts of these compounds with an acid.

7. Composition according to the preceding claim, **characterized in that** the dye precursor is chosen from para-aminophenol oxidation bases, heterocyclic bases and also the addition salts of these compounds with an acid.

8. Composition according to Claim 5, **characterized in that** the dye precursor is chosen from meta-aminophenol couplers, meta-phenylenediamines, meta-diphenols, naphthols, heterocyclic couplers and the addition salts of these compounds with an acid, preferably an aminophenol and/or meta-phenylenediamine coupler.

9. Composition according to any one of the preceding claims, **characterized in that** the oxidizing agent is a peroxide, preferably hydrogen peroxide.

10. Composition according to any one of the preceding claims, in which the alkaline agent is ammonia or an alkanolamine, preferably an alkanolamine.

11. Process for dyeing keratin fibers, **characterized in that** a composition as defined in any one of Claims 1 to 10 is applied to the keratin fibers for a period of time sufficient to develop the desired coloring.

12. Multicompartment device comprising a first compartment which contains a composition comprising the fatty substance(s) other than fatty acids, a second compartment comprising the N,N-bis(β-hydroxyethyl)-para-phenylenediamine(s) and the dye precursor (s) and the alkaline agent and a third compartment comprising the oxidizing agent, it being possible for this third compartment to contain fatty substances; the fatty substance, the dye precursors, the oxidizing agent and the alkaline agent being defined according to any one of the preceding claims.

13. Multicompartment device comprising a first compartment which contains a composition comprising the fatty substance(s) other than fatty acids and one or more oxidizing agents and a second compartment comprising the N,N-bis(β-hydroxyethyl)-para-phenylenediamine(s), the dye precursor(s) and the alkaline agent, the fatty substance, the dye precursor, the oxidizing agent and the alkaline agent being defined according to any one of the preceding claims.

14. Multicompartment device comprising a first compartment containing a composition comprising the fatty substance(s) other than fatty acids, the N,N-bis (β-hydxoxyethyl)-para-phenylenediamine(s), the additional dye(s) and the alkaline agent(s) and a second compartment containing one or more oxidizing agents, the fatty substance, the dye precursor, the oxidizing agent and the alkaline agent being defined according to any one of the preceding claims.

15. Use of a composition as defined in any one of Claims 1 to 10, for the oxidation dyeing of keratin fibers.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- GB 1026978 A
- GB 115 A
- GB 196 A
- DE 2359399
- JP 63169571 A
- JP 3010659 A
- WO 9615765 A
- FR 2750048 A
- DE 3843892
- DE 4133957
- WO 9408969 A
- WO 9408970 A
- FR 2733749 A
- DE 19543988
- FR 2886136 A
- FR 2766177 A
- FR 2766178 A
- FR 2782718 A
- FR 2782716 A
- FR 2782719 A
- FR 2766179 A
- EP 1348695 A
- FR 2586913 A


### Littérature non-brevet citée dans la description


- **WALTER NOLL.** Chemistry and Technology of Silicones. Academie Press, 1968
- Volatile Silicone fluids for cosmetics. Cosmetics and Toiletries. TODD & BYERS, Janvier 1976, vol. 91, 27-32